# EUROPÄISCHE PATENTANMELDUNG

(11) **EP 1 875 878 A1**
(43) Veröffentlichungstag der Anmeldung: **09.01.2008**
(21) Anmeldenummer: 06116235.0
(22) Anmeldetag: 28.06.2006
(51) Int. Cl.: A61C 19/00, A61L 2/18, A61L 2/22

(54) **Verfahren zur Reinigung und Desinfektion von Wasserkanälen von Dentalinstrumenten**

(71) Anmelder: Favodent Karl Huber GmbH, 76229 Karlsruhe (DE)
(72) Erfinder: Wolf, Angela, 76149 Karlsruhe (DE)
(74) Vertreter: Schreiber, Christoph

(57) **Zusammenfassung**

Verfahren zur Reinigung und Desinfektion von Wasserkanälen von Dentalinstrumenten umfassend folgende Schritte:
a) Verbinden der Wasserkanäle des Dentalinstrumentes mit einer ein Desinfektionsmittel und ein Treibmittel enthaltenden Sprühvorrichtung,
b) Durchspülen der Wasserkanäle des Dentalinstrumentes mit dem Desinfektionsmittel aus der Sprühvorrichtung,
c) Einwirken lassen des Desinfektionsmittels.

## Beschreibung

Die vorliegende Erfindung betrifft ein Verfahren zur Reinigung und Desinfektion von Wasserkanälen von Dentalinstrumenten.

In der zahnärztlichen Praxis werden verschiedene Instrumente eingesetzt, die mit Wasser versorgt werden, das aus den dafür vorgesehenen Öffnungen an den Instrumenten austritt.

Typische Instrumente sind Hand- und Winkelstücke oder Turbinen, wie sie beispielsweise in der DE 196 17 670 A1 beschrieben sind.

Das Wasser hat hierbei mehrere Aufgaben. Es dient zum Kühlen der verschiedenen, in die Instrumente eingesetzten Arbeitswerkzeuge, um ein Überhitzen der Zahnsubstanz infolge von Reibungswärme zu vermeiden. Es hat aber zusätzlich auch die Aufgabe, das von den Zähne abgetragene Material wegzuspülen, um den Arbeitsbereich zu säubern.

Typischerweise werden die Hand- und Winkelstücke nach dem Einsatz gereinigt, in dem die Außenflächen mit einem alkoholhaltigen Mittel gereinigt wird.

Das Reinigen und Desinfizieren der in den Instrumenten vorhandenen Wasserkanäle ist schwieriger. Das übliche Verfahren ist ein maschinelles Verfahren zur Thermodesinfektion, in einem Reinigungs- und Desinfektionsgerät (RDG) (ähnlich einer Geschirrspülmaschine). Hierbei muss auf die richtige Beladung, die Vermeidung von Spülschatten, korrekte Temperatur und Zeit und den Einsatz der richtigen Reinigungsmittel geachtet werden. Die Reinigungs- und Desinfektionsgeräte (RDG) müssen über eine Adaption der Kanäle verfügen. Es existiert allerdings keine Überwachung der Kanäle in den RDGs, ob die Durchspülung funktioniert. Entsprechende Reinigungs- und Desinfektionsgeräte sind teuer.

Die Aufgabe der vorliegenden Erfindung war es, zumindest einige der genannten Nachteile des Standes der Technik zu überwinden, insbesondere ein Verfahren bereitzustellen, mit dem in einfacher und kostengünstiger Weise eine Desinfektion der Wasserkanäle möglich ist.

Gelöst wird die Aufgabe durch ein Verfahren zur Reinigung und Desinfektion von Wasserkanälen von Dentalinstrumenten, welches folgende Schritte umfasst:
a) Verbinden der Wasserkanäle des Dentalinstrumentes mit einer ein Desinfektionsmittel und ein Treibmittel enthaltenden Sprühvorrichtung,
b) Durchspülen der Wasserkanäle des Dentalinstrumentes mit dem Desinfektionsmittel aus der Sprühvorrichtung,
c) Einwirken lassen des Desinfektionsmittels.

Erfindungsgemäß kann also die Reinigung der Wasserkanäle mittels Durchspülen mit einem Desinfektionsmittel unter Druck erfolgen, so dass auf den Einsatz teurer Geräte wie eines Reinigungs- und Desinfektionsgerätes verzichtet werden kann.

Hierzu wird eine Sprühvorrichtung, die ein Desinfektionsmittel und ein Treibmittel enthält, mit den Wasserkanälen des Dentalinstrumentes verbunden. Es sind gegebenenfalls Adapter vorgesehen, die eine entsprechende Verbindung zwischen der Sprühvorrichtung und den zu reinigenden Instrumenten ermöglichen. Anschließend erfolgt ein Durchspülen der Wasserkanäle mit dem Desinfektionsmittel.

Das in der Sprühvorrichtung vorhandene Treibmittel treibt hierbei das Desinfektionsmittel aus der Dose durch die Kanäle. Das austretende Desinfektionsmittel wird aufgefangen und entsorgt. Bevorzugt handelt es sich bei der Sprühvorrichtung um eine einfache Sprühdose zur Einmalverwendung. Diese weist einen Anschlussstutzen zum Verbinden mit dem Dentalinstrument auf.

Typischerweise genügt ein Durchspülen für eine Zeit zwischen 1 und 20 s, bevorzugt zwischen 5 und 15 s, insbesondere 6 und 12s. Die genaue Zeit ist abhängig von dem Druck der Sprühvorrichtung.

Anschließend muss das Desinfektionsmittel über einen gewissen Zeitraum einwirken. Diese Einwirkzeit liegt vorzugsweise zwischen 30 s und 15 min, bevorzugte Bereiche liegen zwischen 3 und 10 min, noch mehr bevorzugt 4 und 8 min. Vor dem Einsatz werden die Wasserkanäle vom Desinfektionsmittel befreit, dies erfolgt einfach durch Durchspülen mit Wasser in Trinkwasserqualität.

Als besonders geeignet zur Durchführung des Verfahren hat sich ein Desinfektionsmittel erwiesen, das folgende Zusammensetzung aufweist:
a) 35 bis 50 Gew.-% Ethanol,
b) 5 bis 15 Gew.-% 1-Propanol,
c) 0,03 bis 0,2 Gew.-% einer quaternären Ammoniumverbindung, wie Didecyldimethylammoniumchlorid.

Die Leistungsfähigkeit der Desinfektion scheint auch vom Einsatz des Treibmittels in der Sprühvorrichtung abhängig zu sein. So hat sich Stickstoff als Treibmittel als besonders geeignet erwiesen, da es nicht dazu neigt, aus dem Desinfektionsmittel auszuperlen und dadurch die Benetzung der Fläche zu behindern.

Gegenstand der Vorrichtung ist weiterhin eine Sprühvorrichtung mit einem Desinfektionsmittel enthaltend
a) 35 bis 50 Gew.-% Ethanol,
b) 5 bis 15 Gew.-% 1-Propanol,
c) 0,03 bis 0,2 Gew.-% einer quaternären Ammoniumverbindung, wie Didecyldimethylammoniumchlorid,
und als Treibmittel Stickstoff, sowie die Verwendung der entsprechenden Vorrichtung zur Desinfektion der Wasserkanäle von Dentalinstrumenten, insbesondere Hand- und Winkelstücken und Turbinen.

Es handelt sich um ein validiertes Verfahren mit einer Desinfektionsleistung von mehr als 5 log Einheiten.

Die vorliegende Erfindung wird durch die nachfolgenden Beispiele näher erläutert.

### Beispiel 1

### Herstellung des Desinfektionsmittels

Ein Desinfektionsmittel wurde hergestellt durch Vermischen von 43 Gew.-% Ethanol, 8 Gew.-% 1-Propanol, 0,08 Gew.-% Didecyldimethylammoniumchlorid und 48,92 Gew.-% Wasser. Die ausreichende Desinfektionsleistung einer solchen Zusammensetzung zur Flächendesinfektion ist bekannt. Eine entsprechende Desinfektionslösung wurde in eine Sprühflasche unter Einsatz des Treibmittels Stickstoff abgefüllt.

### Beispiel 2

### Vorbereitung der Hand- und Winkelstücke

Hand- und Winkelstücke sind vom Robert-Koch-Institut (RKI) und dem Bundesinstitut für Arzneimittel und Medizinprodukte (BfArM) in die Risikogruppe Kritisch B eingeordnet. Die Risikogruppe Kritisch B betrifft Medizinprodukte, die die Haut oder Schleimhaut durchdringen oder mit Blut in Kontakt kommen. Aufgrund des komplexen Aufbaus bestehen bei der Gruppe Kritisch B erhöhte Anforderungen an die Aufbereitung. Wegen der schlecht zugänglichen und daher schlecht bespülbaren Oberflächen entsprechender Geräte, ist der Reinigungserfolg nicht visuell überprüfbar.

Entsprechende Hand- und Winkelstücke wurden mit dem Testkeim *Enterococcus hirae* kontaminiert und die mikrobiologische Wirksamkeit in Anlehnung an die DIN EN 1276:1997-08 getestet.

Zum Test wurde ein Hand- und Winkelstück der Firma Kavo Typ INTRAmatic LUX 2 25 LN eingesetzt, dass vor der Überprüfung durch ein thermisches Reinigungs- und Desinfektionsgerät gereinigt und thermisch desinfiziert.

### Beispiel 3

### Bestimmung der Wiederfindungsrate

Es wurde eine Keimsuspension mit *Enterococcus hirae* auf einen Titer von 3,0 x 10⁹KBE/ml eingestellt. Von dieser Lösung wurden 10 µl in das Innere der Wände des Hand- und Winkelstücks (HWS) gespritzt und das HWS anschließend 1 h bei Raumtemperatur getrocknet.

Dann wurden 10 ml Nährbouillon in die Kanäle eingespritzt und in einem sterilen Behälter aufgefangen. Die Nährbouillon wurde wieder mit einer Spritze aufgenommen und nachfolgend noch weitere vier Mal durch die Kanäle gespült.

Anschließend erfolgte eine Keimzahlbestimmung. Dies wird als Wiederfindungsrate bezeichnet. Tabelle 1 zeigt die gefundenen Werte

| Versuch | Ausgangskeimzahl [log] | Endkeimzahl [log] | Verlustrate [log] |
|---|---|---|---|
| 1 | 7,48 | 6,61 | 0,87 |
| 2 | 7,48 | 6,46 | 1,02 |
| 3 | 6,69 | 5,99 | 0,70 |
| 4 | 6,69 | 5,88 | 0,81 |
| 5 | 7,84 | 6,87 | 0,97 |
| Mittelwert | | | 0,87 |

### Beispiel 4

### Bestimmung der Keimreduktion durch das erfindungsgemäße Verfahren

Es wurde eine Keimsuspension von *Enterococcus hirae* eingesetzt. Von dieser Suspension wurden 10 µl in die Wasserkanäle des HWS eingespritzt. Hierbei wurde vom Kopf die Suspension als Tropfen sichtbar. Das HWS wurde 1 h bei Raumtemperatur getrocknet. Nach der Trocknung wurde das HWS mittels der erfindungsgemäßen Sprühvorrichtung mit der Desinfektionslösung 10 s. gespült. Nach einer Einwirkzeit von 5 min erfolgte die mikrobiologische Probennahme.

Hierzu wurden mittels eines Adapters 10 ml Nährbouillon in die Kanäle eingespritzt und in einem sterilen Behälter aufgefangen. Die Nährbouillon wurde wieder mit der Spritze aufgenommen und nachfolgend weitere 4 Mal durch das HWS gespült.

Im Anschluss daran erfolgte die Keimzahlbestimmung.

Es wurden 10 Versuche durchgeführt, wobei die Versuche an 5 Tagen durchgeführt wurden. An jedem Tag wurden mit einer Bakteriensuspension jeweils 2 Kontaminationsversuche durchgeführt, so dass am Ende 5 verschiedene Bakteriensuspensionen mit entsprechenden Ausgangskeimzahlen zum Einsatz kamen.

Tabelle 2 zeigt die Bestimmung der Keimreduktion.

| Versuch | Ausgangskeimzahl [log] | Ausgangskeimzahl nach Korrektur [log] | Keimzahl nach Behandlung [log] | Reduktion [log] |
|---|---|---|---|---|
| 1 | 7,18 | 6,31 | 0 | 6,31 |
| 2 | 7,18 | 6,31 | 0 | 6,31 |
| 3 | 7,34 | 6,47 | 0 | 6,47 |
| 4 | 7,34 | 6,47 | 0 | 6,47 |
| 5 | 6,98 | 6,11 | 0 | 6,11 |
| 6 | 6,98 | 6,11 | 0 | 6,11 |
| 7 | 7,02 | 6,15 | 0 | 6,15 |
| 8 | 7,02 | 6,15 | 0 | 6,15 |
| 9 | 7,21 | 6,34 | 0 | 6,34 |
| 10 | 7,21 | 6,34 | 0 | 6,34 |

Es zeigt sich, dass bei allen durchgeführten Versuchen keine Keime mehr nachgewiesen werden konnten. Die Keimzahlreduktion ist größer als 5 log-Stufen, so dass ein geeignetes Verfahren zur manuell-chemischen Desinfektion von Wasserkanälen von Dentalinstrumenten vorliegt.

## Patentansprüche

1. Verfahren zur Reinigung und Desinfektion von Wasserkanälen von Dentalinstrumenten umfassend folgende Schritte:
a) Verbinden der Wasserkanäle des Dentalinstrumentes mit einer ein Desinfektionsmittel und ein Treibmittel enthaltenden Sprühvorrichtung,
b) Durchspülen der Wasserkanäle des Dentalinstrumentes mit dem Desinfektionsmittel aus der Sprühvorrichtung,
c) Einwirken lassen des Desinfektionsmittels.

2. Verfahren nach Anspruch 1, **dadurch gekennzeichnet, dass** das Desinfektionsmittel folgende Zusammensetzung aufweist:
a) 35 bis 50 Gew.-% Ethanol,
b) 5 bis 15 Gew.-% 1-Propanol,
c) 0,03 bis 0,2 Gew.-% einer quaternären Ammoniumverbindung.

3. Verfahren nach Anspruch 1 oder 2, **dadurch gekennzeichnet, dass** die Sprühvorrichtung als Treibmittel Stickstoff enthält.

4. Verfahren nach mindestens einem der Ansprüche 1 bis 3, **dadurch gekennzeichnet, dass** die Zeit, in der das Desinfektionsmittel durchgespült wird, zwischen 1 und 20 s, bevorzugt 6 und 15 s beträgt.

5. Verfahren nach mindestens einem der Ansprüche 1 bis 4, **dadurch gekennzeichnet, dass** die Zeit, in der das Desinfektionsmittel einwirkt, zwischen 30 s und 15 min bevorzugt 4 bis 8 min liegt.

6. Sprühvorrichtung mit einem Desinfektionsmittel enthaltend eine alkoholische Lösung einer quaternären Ammoniumverbindung und als Treibmittel Stickstoff.

7. Sprühvorrichtung nach Anspruch 6, **dadurch gekennzeichnet, dass** das Desinfektionsmittel
a) 35 bis 50 Gew.-% Ethanol,
b) 5 bis 15 Gew.-% 1-Propanol
c) und 0,03 bis 0,2 Gew.-% einer quaternären Ammoniumverbindung, wie Didecyldimethylammoniumchlorid
enthält.

8. Verwendung einer Sprühvorrichtung nach Anspruch 6 oder 7 zur Desinfektion der Wasserkanäle von Dentalinstrumenten.
